# EUROPEAN PATENT APPLICATION

(11) **EP 4 252 736 A1**
(43) Date of publication of application: **04.10.2023**
(21) Application number: 21898628.9
(22) Date of filing: 25.11.2021
(51) Int. Cl.: A61K 8/02, A61K 8/65, A61K 8/73, A61K 8/81, A61Q 19/00

(54) **METHOD FOR PREPARING MULTILAYER SPHERICAL PARTICLES AND COSMETIC COMPOSITION COMPRISING MULTILAYER SPHERICAL PARTICLES PREPARED THEREBY**

(30) Priority: 26.11.2020 KR 20200161247
(71) Applicant: H&A Pharmachem Co., Ltd, Bucheon-si, Gyeonggi-do 14558 (KR)
(72) Inventor: JI, Hong Geun, Bucheon-si Gyeonggi-do 14600 (KR); PARK, Young Ah, Incheon 21069 (KR); KANG, Yu Jin, Seoul 08767 (KR); KIM, Dong Ock, Seoul 08534 (KR); LEE, Tae Kyung, Daegu 42687 (KR)
(74) Representative: Wichmann, Hendrik
(86) International application number: PCT/KR2021/017466
(87) International publication number: WO 2022/114802

(57) **Abstract**

The present invention relates to a method for preparing multilayer spherical particles and, more specifically, to a method for preparing multilayer spherical particles comprising the steps of: (i) preparing a core component comprising an active ingredient and a shell solution comprising a first polymer component; (ii) forming the core component and shell solution of step (i) into core-shell particles by electro-coextrusion; (iii) drying the core-shell particles obtained from step (ii); and (iv) capsulizing the dried core-shell particles of step (iii) by means of a second polymer. Further, the present invention relates to a cosmetic composition comprising multilayer spherical particles prepared by the method.

## Description

### TECHNICAL FIELD

The present invention relates to a method for preparing multilayered spherical particles. More specifically, the present invention relates to a method for preparing multilayered spherical particles comprising: i) preparing a core component comprising an active ingredient and a shell solution comprising a first polymer; ii) forming core-shell particles by electro-coextrusion of the core component and the shell solution of step (i); iii) drying the core-shell particles obtained in step (ii); and iv) encapsulating the dried core-shell particles of step (iii) with a second polymer.

In addition, the present invention relates to a cosmetic composition comprising multilayered spherical particles prepared by the above method.

### BACKGROUND ART

Recently, as cosmetics have become diversified, consumers have begun to demand aesthetic functions as well as efficacy in cosmetics. As such, cosmetic developers are making efforts to create aesthetically superior products as well as multifunctional, easy-to-use and effective products.

Meanwhile, as one method for delivering active ingredients more stably, there is an encapsulation technology. Encapsulation is a technique in which functional materials are entrapped in a small structure made of polymer to achieve physicochemical stability, volatility and odor suppression, dispersibility improvement, application range expansion through solidification, and toxicity reduction and functional effect increase due to release control. Such an encapsulation structure usually has a capsule form composed of a core-shell or a particle form composed of a core-matrix. Such encapsulation technology has not yet been actively applied in the field of cosmetics.

### DISCLOSURE OF INVENTION

### TECHNICAL PROBLEM

Accordingly, the technical problem of the present invention is the provision of a method for preparing multilayered spherical particles that can efficiently deliver cosmetic ingredients into the skin in a stable form and give excellent aesthetics.

In addition, another technical problem of the present invention is the provision of a cosmetic composition comprising multilayered spherical particles prepared by the above method.

### SOLUTION TO PROBLEM

To solve the above technical problem, the present invention provides a method for preparing multilayered spherical particles comprising:
i) preparing a core component comprising an active ingredient and a shell solution comprising a first polymer;
ii) forming core-shell particles by electro-coextrusion of the core component and the shell solution of step (i);
iii) drying the core-shell particles obtained in step (ii); and
iv) encapsulating the dried core-shell particles of step (iii) with a second polymer.

In addition, to solve another technical problem, the present invention provides a cosmetic composition comprising multilayered spherical particles prepared by the above method.

The present invention is described in detail hereinafter.

According to one aspect to the present invention, there is provided a method for preparing multilayered spherical particles comprising: i) preparing a core component comprising an active ingredient and a shell solution comprising a first polymer; ii) forming core-shell particles by electro-coextrusion of the core component and the shell solution of step (i); iii) drying the core-shell particles obtained in step (ii); and iv) encapsulating the dried core-shell particles of step (iii) with a second polymer.

In step (i) of the method for preparing multilayered spherical particles according to the present invention, a core component comprising an active ingredient and a shell solution comprising a first polymer component are prepared.

In one embodiment according to the present invention, the active ingredient, for example, may be selected from the group consisting of oil, wax, hydrocarbon, higher fatty acid, higher alcohol, silicone, ester and a mixture thereof, but is not limited thereto.

In another embodiment according to the present invention, examples of the oil may include macadamia nut oil, olive oil, jojoba oil, sunflower seed oil, argan oil, camellia oil, avocado oil, soybean oil, grape seed oil, castor oil, rice bran oil and the like, but are not limited thereto.

In another embodiment according to the present invention, examples of the wax may include carnauba wax, candelilla wax, jojoba oil, beeswax, lanolin and the like, but are not limited thereto.

In another embodiment according to the present invention, examples of the hydrocarbon may include liquid paraffin, paraffin, Vaseline^{™} (petroleum jelly), ceresin, microcrystalline wax, squalane and the like, but are not limited thereto.

In another embodiment according to the present invention, examples of the higher fatty acid may include lauric acid, myristic acid, palmitic acid, stearic acid, isostearic acid and the like, but are not limited thereto.

In another embodiment according to the present invention, examples of the higher alcohol may include cetyl alcohol, stearyl alcohol, isostearyl alcohol, 2-octyldodecyl alcohol, diisostearyl malate and the like, but are not limited thereto.

In another embodiment according to the present invention, examples of the silicone may include dimethicone (polydimethylsiloxane), cyclomethicones, silicone polymers, silicone oil and the like, but are not limited thereto.

In another embodiment according to the invention, examples of the ester may include cetyl ethylhexanoate, PEG-20 glyceryl triisostearate, capric/caprylic triglyceride, glyceryl tri(2-ethylhexanoate), isononyl isononanoate, ethylhexyl isononanoate, ethylhexyl palmitate, isostearyl isostearate, neopentyl glycol dicaprate, neopentyl glycol diethylhexanoate, octyldodecyl myristate, pentaerythrityl tetraethylhexanoate, pentaerythrityl tetraisostearate, isotridecyl isononanoate, trimethylopropane triisostearate, squalene and the like, but are not limited thereto.

In another embodiment according to the present invention, as the active ingredient, an oil-soluble cosmetic ingredient-for example, retinol, retinal, glycyrrhizic acid, cica powder, coenzyme Q10, astaxanthin, idebenone, conjugated linoleic acid (CLA), vitamin E (tocopherol), vitamin D, linolenic acid, biotin and the like may be used.

In another embodiment according to the present invention, the active ingredient may form a complex with a covalent organic framework (COF). A covalent organic framework is a two-dimensional or three-dimensional organic solid having an extended structure, in which building blocks are linked by strong covalent bonds. In another embodiment according to the present invention, the active ingredient may form a complex with a covalent organic framework based on, for example, cyclodextrin.

In another embodiment according to the present invention, the first polymer may be selected from the group consisting of nanocellulose, cellulose, hydroxyethyl cellulose, microcrystalline cellulose, starch, polymethyl methacrylate (PMMA), xanthan gum, carbomer, acrylates/C10-30 alkyl acrylate crosspolymer, acacia gum, guar gum, polyvinyl alcohol (PVA), polyvinylpyrrolidone (PVP), gelatin, hyaluronic acid, collagen, pullulan, polyglycolide (PGA), agar, gum arabic, carrageenan gum, gellan gum, karaya gum, methylcellulose, locust bean gum, alginate, sodium alginate, glucomannan, succinyl chitosan, pullulan lactide, chitosan, polyethylene glycol (PEG)-polycaprolactone (PCL)-polyethylene glycol (PEG) triblock copolymer, poloxamer 407 (Pluronic^{™} F-127), acetyl hydroxypropyl cellulose and a mixture thereof, but is not limited thereto.

In another embodiment according to the present invention, as the first polymer a mixture of two or more types of polymers may be used.

In another embodiment according to the present invention, as the first polymer a mixture of three or more types of polymers may be used.

In another embodiment according to the present invention, the concentration of the first polymer in the shell solution may be 1 to 50% by weight, 2 to 45% by weight or 5 to 40% by weight.

In step (ii) of the method for preparing multilayered spherical particles according to the present invention, core-shell particles are formed by electro-coextrusion of the core component comprising an active ingredient and the shell solution comprising a first polymer.

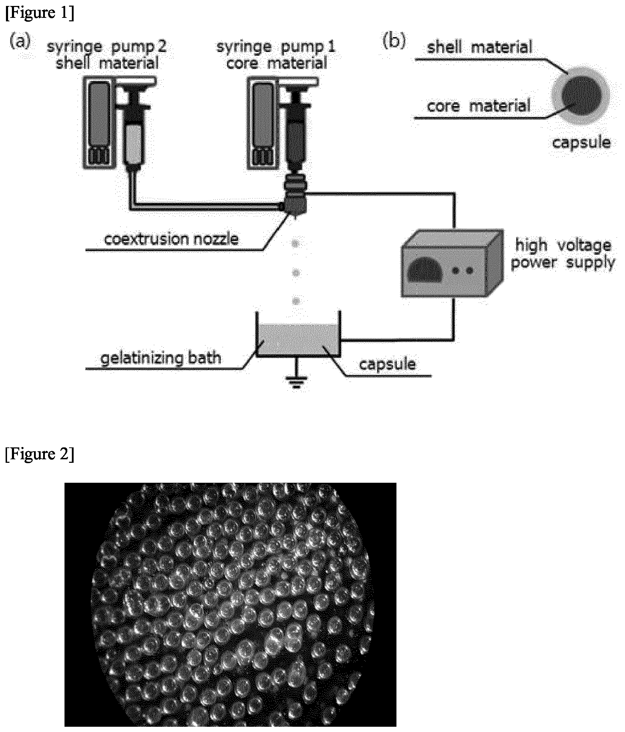

In electro-coextrusion, different materials are expelled from two coaxial capillaries under an electric field to form a core-shell capsule (Figure 1). Because the effect of the characteristics of the core material (core) to be entrapped is small, it can be easily applied to the encapsulation of various functional materials or mixtures thereof, and the encapsulation efficiency is excellent.

In another embodiment according to the present invention, the electro-coextrusion of the core component may be carried out at a flow rate of 0.1 to 10 mL/min, 0.2 to 8 mL/min or 0.5 to 5 mL/min.

In another embodiment according to the present invention, the electro-coextrusion of the shell solution may be carried out at a flow rate of 0.5 to 20 mL/min, 1 to 15 mL/min or 3 to 12 mL/min.

In another embodiment according to the present invention, the electro-coextrusion may be carried out at 1,000 to 5,000 volts (V), 1,500 to 4,000 volts (V) or 2,000 to 3,000 volts (V).

In another embodiment according to the present invention, a calcium chloride solution may be used as a curing agent in the electro-coextrusion.

In step (iii) of the method for preparing multilayered spherical particles according to the present invention, the core-shell particles obtained in step (ii) are dried.

In another embodiment according to the present invention, drying of the core-shell particles may be carried out at 60 to 90°C.

In step (iv) of the method for preparing multilayered spherical particles according to the present invention, the dried core-shell particles obtained in step (iii) are encapsulated with a second polymer to obtain multilayered spherical particles.

The encapsulation with the second polymer may be carried out by a method known in the art and there is no particular limitation thereto.

In another embodiment according to the present invention, the second polymer may be polymers that can act as cosmetic ingredients-for example, may be selected from the group consisting of collagen, hyaluronic acid, pullulan, proteoglycan, beta-glucan, glucan, polyglutamic acid, chitosan and a mixture thereof, but is not limited thereto.

In another embodiment according to the present invention, the multilayered spherical particles prepared by the above method may have a diameter of 200 to 1,500 µm.

According to another aspect of the present invention, there is provided a cosmetic composition comprising multilayered spherical particles prepared by the above method.

In the present invention, the cosmetic composition may be formulated into various products such as face lotion, emulsion, body lotion, cream, essence, ampoule and

BB (blemish balm) cream, but is not limited thereto. In the present invention, the cosmetic composition may include various amounts of multilayered spherical particles according to the needs of the formulation-for example, the multilayered spherical particles may be comprised in an amount of 0.1 to 50% by weight.

### EFFECTS OF INVENTION

According to the present invention, multilayered spherical particles can be prepared with excellent encapsulation efficiency and high production efficiency of various active ingredients. The multilayered spherical particles prepared according to the present invention can not only efficiently deliver active ingredients into the skin in a stable form, but also have good appearance to give excellent aesthetic sensibility.

### BRIEF DESCRIPTION OF DRAWINGS

Figure 1 is a schematic diagram of electro-coextrusion apparatus.
Figure 2 is an enlarged photograph of multilayered spherical particles prepared in Example 1.
Figure 3 is a result of measuring the particle size of multilayered spherical particles prepared in Example 1.

### MODE FOR THE INVENTION

Hereinafter, the present invention is explained in more detail with the following examples. However, it must be understood that the protection scope of the present invention is not limited to the examples.

### Example 1: Preparation of multilayered spherical particles using gelatin/acacia gum/carbomer

A shell solution was prepared from Ingredient A according to the composition recited in Table 1, and a solution was prepared by dissolving a core component of Ingredient B and 1% calcium chloride as a curing agent in purified water. Then, electro-coextrusion was carried out with the above solutions by the use of Encapsulator B- 390 (Buchi, Switzerland) under the following conditions to obtain core-shell particles.
- Nozzle: 150 µm (core), 400 µm (shell)
- Flow rate: 1.5 mL/min (core), 10 mL/min (shell)
- Frequency: 600 Hz
- Pressure: 0.5 bar
- Amplitude: 3
- Charge: 2,300 V

After drying the obtained core-shell particles at 80°C for 12 hours, the dried core-shell particles were put in a container, and Ingredient C was slowly added thereto. Then, encapsulation was carried out by mixing them with a winged disperser for 10 minutes to prepare multilayered spherical particles.

**[Table 1]**

| **Ingredient** | | **Content (parts by weight)** |
|---|---|---|
| A | Gelatin | 2.5 |
| | Acacia gum | 1.5 |
| | Carbomer | 0.3 |
| | Dipropylene glycol | 15 |
| | Glycerin | 6 |
| | Butylene glycol | 12 |
| | Purified water | 15 |
| B | Retinol/capric/caprylic triglyceride | 90 |
| C | Chitosan (2% solution) | 50 |

### Example 2: Preparation of multilayered spherical particles using alginate/nanocellulose/carbomer

Multilayered spherical particles were prepared by the same method as described in Example 1 with the constitutional composition of Table 2.

**[Table 2]**

| **Ingredient** | | **Content (parts by weight)** |
|---|---|---|
| A | Alginate | 2 |
| | Nanocellulose | 12 |
| | Carbomer | 0.3 |
| | Dipropylene glycol | 12 |
| | Glycerin | 8 |
| | Butylene glycol | 15 |
| | Purified water | 15 |
| B | Olive oil | 35 |
| C | Collagen (10% solution) | 40 |

### Example 3: Preparation of multilayered spherical particles using aleinate/acacia gum/carrageenan gum

Multilayered spherical particles were prepared by the same method as described in Example 1 with the constitutional composition of Table 3.

**[Table 3]**

| **Ingredient** | | **Content (parts by weight)** |
|---|---|---|
| A | Alginate | 3 |
| | Acacia gum | 0.8 |
| | Carrageenan gum | 1 |
| | Dipropylene glycol | 15 |
| | Glycerin | 12 |
| | Butylene glycol | 8 |
| | Purified water | 15 |
| B | Candelilla wax | 40 |
| C | Hyaluronic acid (1% solution) | 50 |

### Example 4: Preparation of multilayered spherical particles using xanthan gum/PVA/carbomer

Multilayered spherical particles were prepared by the same method as described in Example 1 with the constitutional composition of Table 4.

**[Table 4]**

| **Ingredient** | | **Content (parts by weight)** |
|---|---|---|
| A | Xanthan gum | 3 |
| | Polyvinyl alcohol | 4 |
| | Carbomer | 0.5 |
| | Dipropylene glycol | 12 |
| | Glycerin | 8 |
| | Butylene glycol | 10 |
| | Purified water | 18 |
| B | Paraffin | 38 |
| C | Pullulan (10% solution) | 40 |

### Example 5: Preparation of multilayered spherical particles using PVP/guar gum/acrylate/C10-30 alkyl acrylate crosspolymer

Multilayered spherical particles were prepared by the same method as described in Example 1 with the constitutional composition of Table 5.

**[Table 5]**

| **Ingredient** | | **Content (parts by weight)** |
|---|---|---|
| A | Polyvinylpyrrolidone | 1.5 |
| | Guar gum | 3 |
| | Acrylates/C10-30 alkyl acrylate crosspolymer | 0.8 |
| | Dipropylene glycol | 12 |
| | Glycerin | 10 |
| | Butylene glycol | 12 |
| | Purified water | 18 |
| B | Palmitic acid | 40 |
| C | Proteoglycan (10% solution) | 40 |

### Example 6: Preparation of multilayered spherical particles using gum arabic/gellan gum/locust bean gum

Multilayered spherical particles were prepared by the same method as described in Example 1 with the constitutional composition of Table 6.

**[Table 6]**

| **Ingredient** | | **Content (parts by weight)** |
|---|---|---|
| A | Gum arabic | 2 |
| | Gellan gum | 1.5 |
| | Locust bean gum | 0.3 |
| | Dipropylene glycol | 15 |
| | Glycerin | 6 |
| | Butylene glycol | 12 |
| | Purified water | 15 |
| B | Stearyl alcohol | 35 |
| C | Glucan (10% solution) | 40 |

### Example 7: Preparation of multilayered spherical particles using glucomannan/gellan gum/carbomer

Multilayered spherical particles were prepared by the same method as described in Example 1 with the constitutional composition of Table 7.

**[Table 7]**

| **Ingredient** | | **Content (parts by weight)** |
|---|---|---|
| A | Glucomannan | 3 |
| | Gellan gum | 1.2 |
| | Carbomer | 0.2 |
| | Dipropylene glycol | 15 |
| | Glycerin | 6 |
| | Butylene glycol | 12 |
| | Purified water | 15 |
| B | Dimethicone | 38 |
| C | Polyglutamic acid (10% solution) | 40 |

### Example 8: Preparation of multilayered spherical particles using succinyl chitosan/pullulan lactide/carbomer

Multilayered spherical particles were prepared by the same method as described in Example 1 with the constitutional composition of Table 8.

**[Table 8]**

| **Ingredient** | | **Content (parts by weight)** |
|---|---|---|
| A | Succinyl chitosan | 3 |
| | Pullulan lactide | 1.5 |
| | Carbomer | 0.5 |
| | Dipropylene glycol | 15 |
| | Glycerin | 12 |
| | Butylene glycol | 8 |
| | Purified water | 20 |
| B | Cyclodextrin-COF/olive oil | 60 |
| C | Polyglutamic acid (10% solution) | 40 |

### Example 9: Preparation of multilayered spherical particles using succinyl chitosan/PEG-PCL-PEG/carbomer

Multilayered spherical particles were prepared by the same method as described in Example 1 with the constitutional composition of Table 9.

**[Table 9]**

| **Ingredient** | | **Content (parts by weight)** |
|---|---|---|
| A | Succinyl chitosan | 2 |
| | PEG-PCL-PEG triblock copolymer | 1.5 |
| | Carbomer | 0.4 |
| | Dipropylene glycol | 12 |
| | Glycerin | 8 |
| | Butylene glycol | 12 |
| | Purified water | 18 |
| B | Cyclodextrin-COF/retinol/soybean oil | 40 |
| C | Polyglutamic acid (10% solution) | 40 |

### Example 10: Preparation of multilayered spherical particles using succinyl chitosan/nanocellulose/carbomer

Multilayered spherical particles were prepared by the same method as described in Example 1 with the constitutional composition of Table 10.

**[Table 10]**

| **Ingredient** | | **Content (parts by weight)** |
|---|---|---|
| A | Succinyl chitosan | 2 |
| | Nanocellulose | 1.8 |
| | Carbomer | 0.5 |
| | Dipropylene glycol | 12 |
| | Glycerin | 8 |
| | Butylene glycol | 10 |
| | Purified water | 18 |
| B | Cyclodextrin-COF/retinol/soybean oil | 45 |
| C | Polyglutamic acid (10% solution) | 40 |

### Example 11: Preparation of multilayered spherical particles using succinyl chitosan/acetyl hydroxypropyl cellulose/carbomer

Multilayered spherical particles were prepared by the same method as described in Example 1 with the constitutional composition of Table 11.

**[Table 11]**

| **Ingredient** | | **Content (parts by weight)** |
|---|---|---|
| A | Succinyl chitosan | 3 |
| | Acetyl hydroxypropyl cellulose | 2 |
| | Carbomer | 0.3 |
| | Dipropylene glycol | 12 |
| | Glycerin | 6 |
| | Butylene glycol | 12 |
| | Purified water | 20 |
| B | Cyclodextrin-COF/cica powder/soybean oil | 38 |
| C | Polyglutamic acid (10% solution) | 40 |

### Example 12: Preparation of multilayered spherical particles using succinyl chitosan/poloxamer 407/carbomer

Multilayered spherical particles were prepared by the same method as described in Example 1 with the constitutional composition of Table 12.

**[Table 12]**

| **Ingredient** | | **Content (parts by weight)** |
|---|---|---|
| A | Succinyl chitosan | 2 |
| | Poloxamer 407 | 1.5 |
| | Carbomer | 0.3 |
| | Dipropylene glycol | 15 |
| | Glycerin | 6 |
| | Butylene glycol | 12 |
| | Purified water | 15 |
| B | Cyclodextrin-COF/oil-soluble glycyrrhizic acid/ soybean oil | 42 |
| C | Collagen (10% solution) | 40 |

### Example 13 and Comparative Example: Preparation of lotions with and without multilayered spherical particles

With the constitutional composition of Table 13, each ingredient was introduced into a container and dissolved at a temperature of 80°C, then mixed for 5 minutes using a homo-mixer, cooled and deaerated to obtain a lotion.

**[Table 13]**

| **Ingredient** | **Example 13 (% by weight)** | **Comparative Example (% by weight)** |
|---|---|---|
| Multilayered spherical particles of Example 1 | 10 | - |
| Retinol | - | 2.5 |
| Polyglyceryl-3 stearate/citrate | 2 | 2 |
| Cetearyl alcohol | 0.5 | 0.5 |
| Olive oil | 5 | 5 |
| Capric/caprylic triglyceride | 4 | 4 |
| Macadamia nut oil | 6 | 6 |
| Glycerin | 5 | 5 |
| Carbopol | 0.2 | 0.2 |
| Purified water | 67.3 | 74.8 |
| Total | 100 | 100 |

### Example 14: Preparation of body lotion containing multilayered spherical particles

With the constitutional composition of Table 14, each ingredient was introduced into a container and dissolved at a temperature of 80°C, then mixed for 5 minutes using a homo-mixer, cooled and deaerated to obtain a body lotion.

**[Table 14]**

| **Ingredient** | **Content (% by weight)** |
|---|---|
| Multilayered spherical particles of Example 2 | 5 |
| Polyglyceryl-3 methylglucose distearate | 3 |
| Phytosqualane | 5 |
| Capric/caprylic triglyceride | 2 |
| Sunflower oil | 5 |
| Olive oil | 5 |
| Glycerin | 8 |
| Purified water | 69 |
| Total | 100 |

### Example 15: Preparation of cream containing multilayered spherical particles

With the constitutional composition of Table 15, each ingredient was introduced into a container and dissolved at a temperature of 80°C, then mixed for 5 minutes using a homo-mixer, cooled and deaerated to obtain a cream.

**[Table 15]**

| **Ingredient** | **Content (% by weight)** |
|---|---|
| Multilayered spherical particles of Example 3 | 6 |
| Sorbitan stearate/sucrose cocoate | 5 |
| Cetearyl alcohol | 1.5 |
| Phytosqualane | 3 |
| Olive oil | 3 |
| Soybean oil | 2 |
| Glycerin | 8 |
| Carbopol | 0.36 |
| Purified water | 71.14 |
| Total | 100 |

### Example 16: Preparation of essence containing multilayered spherical particles

With the constitutional composition of Table 16, each ingredient was introduced into a container and dissolved at a temperature of 80°C., then mixed for 5 minutes using a homo-mixer, cooled and deaerated to obtain an essence.

**[Table 16]**

| **Ingredient** | **Content (% by weight)** |
|---|---|
| Multilayered spherical particles of Example 4 | 20 |
| Carbopol | 0.4 |
| Hyaluronic acid | 1 |
| Coenzyme Q10 | 0.1 |
| Sodium EDTA | 0.05 |
| Calcium hydroxide | 0.03 |
| PEG-1500 | 3 |
| Glycerin | 7 |
| Purified water | 68.42 |
| Total | 100 |

### Example 17: Preparation of transparent essence containing multilayered spherical particles

With the constitutional composition of Table 17, each ingredient was introduced into a container and dissolved at a temperature of 80°C, then mixed for 5 minutes using a homo-mixer, cooled and deaerated to obtain a transparent essence.

**[Table 17]**

| **Ingredient** | **Content (% by weight)** |
|---|---|
| Multilayered spherical particles of Example 5 | 10 |
| Carbopol | 0.4 |
| Lubrajel^{™} | 20 |
| Glutamic acid | 5 |
| Sodium EDTA | 0.05 |
| Calcium hydroxide | 0.03 |
| PEG-1500 | 3 |
| Glycerin | 7 |
| Purified water | 54.52 |
| Total | 100 |

### Example 18: Preparation of BB cream containing multilayered spherical particles

Ingredient A was prepared by three (3)-time treatment with a triple roller mill device. Ingredient B was put into the manufacturing section and heated to 75 to 80°C, and then Ingredient A treated with a triple roller mill device was added thereto and dispersed. Ingredient C was dissolved in a separate container, heated to 80 to 85°C while stirring with a homo-mixer. Ingredient C was added to the above prepared ingredients, and the mixture was emulsified for 10 minutes. After emulsification was completed, the obtained product was cooled to 45°C while stirring using a stirrer, then cooled to 25°C again, and then put in a container and matured.

**[Table 18]**

| **Ingredient** | | **Content (% by weight)** |
|---|---|---|
| A | Phenyl trimethicone | 12 |
| | Titanium dioxide | 6.2 |
| | Mica | 1 |
| | Dimethylpolysiloxane | 0.3 |
| | Iron oxide | 0.5 |
| B | Beeswax | 2 |
| | Cetyl PEG/PPG-10/1 dimethicone | 1.5 |
| | Capric/caprylic triglyceride | 3 |
| | Preservative | 0.01 |
| C | Multilayered spherical particles of Example 7 | 5 |
| | Disodium EDTA | 0.05 |
| | Butylene glycol | 5 |
| | Glycerin | 4 |
| | Purified water | 59.44 |
| Total | | 100 |

### Experimental Example 1: Measurement of particle size of multilayered spherical particles

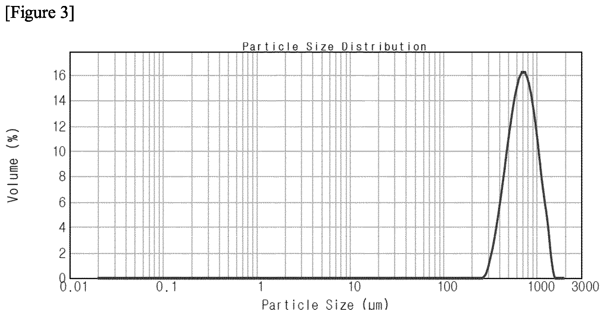

The particle size of the multilayered spherical particles prepared in Example 1 was measured by the use of a particle size analyzer (Mastersizer 2000, MLAVERN, UK), and the result is represented in Figure 3. From the result of the measurement, it can be known that the particle size is 438 to 1,092 µm.

### Experimental Example 2: Measurement of changes in retinol content

The change in retinol content in the lotions of Example 13 and Comparative Example was quantitatively analyzed at 7-day intervals for one month while maintaining light-shielding conditions at 25°C and light-exposure conditions at 40°C. As an equipment HPLC (Waters, USA) was used, and the analysis conditions were as follows. The analyzed results are represented in Table 19, and light-exposure conditions at 40°C are described in parentheses.
- Detection: UV-Spectrophotometer (325nm)
- Column: C18 (3.9 × 150 mm)
- Flow rate: 1.0 mL/min
- Mobile phase: Methanol: H₂O (90 : 10)

**[Table 19]**

| Days | 0 | 7 | 13 | 22 | 29 | 35 |
|---|---|---|---|---|---|---|
| Example 13 | 100 (100) | 98 (99) | 97 (95) | 94 (92) | 92 (88) | 90 (86) |
| Comparative Example | 100 (100) | 90 (90) | 85 (84) | 65 (60) | 60 (32) | 45 (20) |

| | | | | | | |
|---|---|---|---|---|---|---|
| (Retinol Residual Content%) | | | | | | |

### Experimental Example 3: Test for effect on promoting transdermal absorption

The artificial skin, Neoderm (Tego Science, Korea) was mounted to a Franz-type diffusion cell (Lab Fine Instruments, Korea). 50 mM phosphate buffer (pH 7.4, 0.1M NaCl) was added to a receptor cell (5 ml) of the Franz-type diffusion cell. A diffusion cell was then mixed and diffused at 600 rpm, 32°C, and 50 µl of the lotions of Example 13 and Comparative Example, respectively, were added to donor cells. Absorption and diffusion were carried out according to the predetermined time, and the area of the skin where the absorption and diffusion were carried out was 0.64 cm². After finishing the absorption and diffusion of the active ingredient, the residues-which were not absorbed and remained on the skin-were cleaned with dried Kimwipes^{™} or 10 ml of ethanol. The skin in which the active ingredient was absorbed and diffused was homogenized by the use of a tip-type homogenizer, and retinol absorbed into the skin was then extracted with 4 ml of dichloromethane. The extract was then filtrated with a 0.45 µm nylon membrane filter. The content of retinol was measured by high-performance liquid chromatography (HPLC) with the following conditions, and the results are represented in Table 20.

**[Table 20]**

| | Transdermal absorption (µg) | Rate of increase |
|---|---|---|
| Lotion of Example 13 | 3.213 | 2-fold |
| Lotion of Comparative Example | 1.5121 | - |
| A) Column: C18 (3.9 × 150 mm, 5 µm) | | |
| B) Mobile phase: methanol: water = 90 : 10 | | |
| C) Flow rate: 0.8 mL/min | | |
| D) Detector: UV 325 nm | | |

As can be seen from Table 20, it was confirmed that the lotion according to the present invention has superior transdermal absorption compared to the general lotion.

### Experimental Example 4: Measurement of skin moisturizing ability

After applying the lotions of Example 13 and Comparative Example, the moisturizing ability was measured by Comeometer CM850 (Courage + Khazaka electronic GmbH, Germany), and the results are represented in Table 21. As can be seen from Table 21, it can be known that the lotion containing the multilayered spherical particles has superior moisturizing ability than the lotion without them.

**[Table 21]**

| Days | 0 | 3 | 7 | 14 | 29 |
|---|---|---|---|---|---|
| Lotion of Example 13 | 65 | 100 | 97 | 95 | 90 |
| Lotion of Comparative Example | 65 | 95 | 90 | 85 | 75 |
| Untreated | 65 | 75 | 74 | 73 | 70 |

| | | | | | |
|---|---|---|---|---|---|
| (Corneometer/a.u.) | | | | | |

## Claims

1. A method for preparing multilayered spherical particles comprising:
i) preparing a core component comprising an active ingredient and a shell solution comprising a first polymer;
ii) forming core-shell particles by electro-coextrusion of the core component and the shell solution of step (i);
iii) drying the core-shell particles obtained in step (ii); and
iv) encapsulating the dried core-shell particles of step (iii) with a second polymer.

2. The method for preparing multilayered spherical particles according to Claim 1, wherein the active ingredient of step (i) is selected from the group consisting of oil, wax, hydrocarbon, higher fatty acid, higher alcohol, silicone, ester and a mixture thereof.

3. The method for preparing multilayered spherical particles according to Claim 2, wherein the active ingredient of step (i) forms a complex with a covalent organic framework (COF).

4. The method for preparing multilayered spherical particles according to Claim 1, wherein the first polymer of step (i) is selected from the group consisting of nanocellulose, cellulose, hydroxyethyl cellulose, microcrystalline cellulose, starch, polymethyl methacrylate (PMMA), xanthan gum, carbomer, acrylates/C10-30 alkyl acrylate crosspolymer, acacia gum, guar gum, polyvinyl alcohol (PVA), polyvinylpyrrolidone (PVP), gelatin, hyaluronic acid, collagen, pullulan, polyglycolide (PGA), agar, gum arabic, carrageenan gum, gellan gum, karaya gum, methylcellulose, locust bean gum, alginate, sodium alginate, glucomannan, succinyl chitosan, pullulan lactide, chitosan, polyethylene glycol (PEG)-polycaprolactone (PCL)-polyethylene glycol (PEG) triblock copolymer, poloxamer 407, acetyl hydroxypropyl cellulose and a mixture thereof.

5. The method for preparing multilayered spherical particles according to Claim 1, wherein the first polymer in the shell solution of step (i) has a concentration of 1 to 50% by weight.

6. The method for preparing multilayered spherical particles according to Claim 1, wherein the electro-coextrusion of the core component in step (ii) is carried out at a flow rate of 0.1 to 10 mL/min.

7. The method for preparing multilayered spherical particles according to Claim 1, wherein the electro-coextrusion of the shell solution in step (ii) is carried out at a flow rate of 0.5 to 20 mL/min.

8. The method for preparing multilayered spherical particles according to Claim 1, wherein the electro-coextrusion of step (ii) is carried out at 1,000 to 5,000 volts (V).

9. The method for preparing multilayered spherical particles according to Claim 1, wherein a calcium chloride solution is used as a curing agent in the electro-coextrusion of step (ii).

10. The method for preparing multilayered spherical particles according to Claim 1, wherein the drying of step (iii) is carried out at 60 to 90°C.

11. The method for preparing multilayered spherical particles according to Claim 1, wherein the second polymer of step (iv) is selected from the group consisting of collagen, hyaluronic acid, pullulan, proteoglycan, beta-glucan, glucan, polyglutamic acid, chitosan and a mixture thereof.

12. The method for preparing multilayered spherical particles according to Claim 1, wherein the prepared multilayered spherical particles have a diameter of 200 to 1,500 µm.

13. A cosmetic composition comprising the multilayered spherical particles prepared by the method according to any one of Claims 1 to 12.

14. The cosmetic composition according to Claim 13, which comprises 0.1 to 50% by weight of the multilayered spherical particles.
